**Europäisches Patentamt**

**European Patent Office**

**Office européen des brevets**

(19)

(11) **EP 1 038 855 A1**

(12) # EUROPEAN PATENT APPLICATION

(43) Date of publication:
**27.09.2000 Bulletin 2000/39**

(51) Int Cl.7: **C07C 2/86**, B01J 19/08

(21) Application number: **00810248.5**

(22) Date of filing: **23.03.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **24.03.1999 EP 99810261**

(71) Applicant: **ABB RESEARCH LTD.**
**8050 Zürich (CH)**

(72) Inventors:
• **Eliasson, Baldur, Dr.**
**5413 Birmenstorf (CH)**

• **Zhang, Kui, Dr.**
**5405 Baden-Dättwil (CH)**
• **Kogelschatz, Ulrich, Dr.**
**5212 Hausen (CH)**
• **Killer, Eric**
**5430 Wettingen (CH)**

(74) Representative: **ABB Patent Attorneys**
**c/o ABB Business Services Ltd,**
**Intellectual Property (SLE-I),**
**Haselstrasse 16/699**
**5401 Baden (CH)**

(54) **Fuel synthesis by electric barrier discharge process**

(57)     A method of transforming a normally gaseous composition containing carbon dioxide and at least one normally gaseous hydrocarbon into a product stream containing at least one normally liquid hydrocarbon; the method comprising the steps of feeding the composition into a reactor including a first electrode means, a second electrode means and at least one layer of a normally solid dielectric material positioned between the first and the second electrode means; submitting the composition within the reactor in the presence of a normally solid catalyst to a dielectric barrier discharge, wherein the normally solid catalyst is a member selected from the group of zeolites, modified zeolites and zeolite-like materials, and wherein the normally solid catalyst has an at least partially crystalline solid structure with at least one 10-ring channel; and controlling the dielectric barrier discharge to convert the normally gaseous composition into the product stream.

FIG. 1

EP 1 038 855 A1

**Description**

Background of the Invention

The present invention relates to a method of transforming a normally gaseous composition containing carbon dioxide and at least one normally gaseous hydrocarbon into a product stream containing at least one normally liquid hydrocarbon, furthermore the present invention relates to a normally liquid fuel and to an apparatus for transforming a normally gaseous composition containing carbon dioxide and at least one normally gaseous hydrocarbon into a product stream containing at least one normally liquid hydrocarbon.

One of the major problems facing mankind is the global warming of the atmosphere due to man-made emissions of greenhouse gases such as carbon dioxide, methane, chlorofluorocarbons, nitrous oxide or ozone. One possible approach to mitigate the emissions of these greenhouse gases to the atmosphere would be to recycle them in a chemical process to form useful products. Among all the man-made greenhouse gases, methane and carbon dioxide contribute to most of the greenhouse effect.

Prior Art

There are two general pathways to upgrade methane being the principal constituent of natural gas into higher hydrocarbons. First, by way of indirect conversion mainly requiring syngas production in a first step, and second, by way of direct conversion. A major difficulty, in particular for direct methane conversion, is the high strength of the C-H bonds in the methane molecule causing methane to be a very stable molecule and its reactions to have a high activation energy. There are many ways to activate methane such as photochemical and electrochemical activation, laser-induced activation, and radiolysis, as well as catalytic or even thermal activation. The direct methane conversion methods include, e.g. oxidative coupling, partial oxidation to methanol and formaldehyde, non-oxidative routes and organometallic routes. However, the direct methane transformations into higher hydrocarbons show, so far, poor economics, low conversions and low yields making them not suitable for practical applications.

A great effort has also been given to chemical fixation of carbon dioxide. Heterogeneous catalysis has been considered to be a desirable route for carbon dioxide utilization. But a large amount of additional energy or expensive hydrogen is required for conventional catalytic utilization of carbon dioxide since the carbon dioxide molecule has a very low energy content. There is still no confirmed technology by far for utilizing such a plentiful carbon source.

A few processes for the synthesis of liquid fuel, such as normally liquid hydrocarbons, starting from gaseous compositions are known, such as the "Mobil process" and the "Fischer-Tropsch process" schematically shown in equation (1) and (2).

$$CO + H_2 \rightarrow CH_3OH \rightarrow gasoline \qquad (1)$$

$$CO + H_2 \rightarrow gasoline \qquad (2)$$

For both heterogeneous catalyzed processes the production of "synthesis gas", a mixture of CO and $H_2$ also named "syngas", represents the first step along the path to methanol and gasoline respectively. Even if the "Mobil process" (eq. (1)) and the "Fischer-Tropsch process" (eq. (2)) are practiced today for industrial fuel synthesis production, e.g in South Africa, Malaysia and New Zealand, they are non-economic "political processes", heavily supported by governmental subsidies. The lack of profitableness is either due to the usually required high pressures at which the processes take place as well as to the high production costs of syngas and the fact that the produced syngas needs to be compressed before applied in the processes (1) and (2). Thus, about 60% to 80% of the total cost of the processes (1) and (2) goes to production and compression of syngas.

The industrial production of syngas mostly derives from the energy-intensive steam reforming of methane shown in equation (3):

$$H_2O + CH_4 \rightarrow CO + 3\,H_2 \qquad \Delta H° = 206.1 \text{ kJ/mol} \qquad (3)$$

Syngas can also be produced from the greenhouse gases methane and carbon dioxide as shown in equation (4). However, such a reforming of carbon dioxide by methane is also a very energy-intensive process and requires high temperatures. Moreover, deposition of carbon on the catalyst always causes problems for this reaction.

$$CO_2 + CH_4 \rightarrow 2\,CO + 2\,H_2 \qquad \Delta H^\circ = 258.9\ kJ/mol \qquad\qquad (4)$$

Non-equilibrium plasma chemical processes occuring in the volume part of electrical non-equilibrium discharges have attracted a great deal of interest. Particularly, silent gas discharges have demonstrated their suitability for large-scale industrial applications, such as the generation of ozone. A characteristic of the silent discharge is the presence of a dielectric. Therefore silent gas discharges are also referred to as dielectric barrier discharges.

Recently, the utilization of greenhouse gases for the synthesis of methanol or methane in such silent gas discharge reactors has also been described. Thus, DE 42 20 865 describes a method and an apparatus for the hydrogenation of carbon dioxide leading in particular to methane or methanol by exposing a mixture of carbon dioxide and a substance containing hydrogen atoms, preferably hydrogen or water, to a dielectric barrier discharge. An overview of the progress in this field have been summarized by Bill et al. in Energy Conversion Management 38 (1997) 415 (this report being incorporated herein for all purposes by way of reference). It is noteworthy, however, that the reported maximum yield of methanol was only about 1%.

Objects of the Invention

Accordingly, it is an object of the present invention to provide for a method of transforming a normally gaseous composition into a product stream containing at least one normally liquid hydrocarbon, which method minimizes or even avoids the disadvantages of the prior art.

Moreover, it is an object of the present invention to provide for a method of transforming a normally gaseous composition into a product stream containing at least one normally liquid hydrocarbon, which method can be carried out at low pressures, and preferably at low temperatures.

It is another object of the present invention to provide for a method of producing liquid fuel and gasoline, respectively, from carbon dioxide and at least one normally gaseous hydrocarbon in reasonable yields and in a direct manner, i.e. making the expensive formation of syngas no longer necessary.

It is a further object of the present invention to provide for a method of producing liquid fuel and gasoline, respectively, from carbon dioxide and at least one normally gaseous hydrocarbon in reasonable yields and in a direct manner, which gasoline is rich in C6 to C10 hydrocarbons.

Another object of the present invention is to provide for an apparatus that allows the transformation of a gaseous composition containing carbon dioxide and at least one normally gaseous hydrocarbon into a product stream ccontaining at least one normally liquid hydrocarbon.

Further objects and advantages of the present invention will become apparent as this specification proceeds.

Brief Summary of the Invention

We have found that the objects can be achieved according to a first general embodiment of the invention by a method as set forth in claim 1. Accordingly, the invention provides for a method of transforming a normally gaseous composition containing carbon dioxide and at least one normally gaseous hydrocarbon into a product stream containing at least one normally liquid hydrocarbon; said method comprising the steps of feeding said composition into a reactor including a first electrode means, a second electrode means and at least one layer of a normally solid dielectric material positioned between said first and said second electrode means; submitting said composition within said reactor in the presence of a normally solid catalyst to a dielectric barrier discharge, wherein said normally solid catalyst is a member selected from the group of zeolites, modified zeolites and zeolite-like materials, and wherein said normally solid catalyst has an at least partially crystalline solid structure with at least one 10-ring channel; and controlling said dielectric barrier discharge to convert said normally gaseous composition into said product stream.

In a second general embodiment the invention provides for a normally liquid fuel obtainable by a dielectric barrier discharge, the normally liquid fuel comprising at least 60 mol% of hydrocarbons having a normal boiling range of between about 50°C and about 210°C, and less than 40 mol% of oxygenated hydrocarbons.

In a third general embodiment the invention provides for an apparatus for transforming a normally gaseous composition containing carbon dioxide and at least one normally gaseous hydrocarbon into a product stream containing at least one normally liquid hydrocarbon as set forth in claim 11.

Definitions, Detailed Description of Preferred Embodiments and Elements of the Invention

The term "about" as used herein before any numeral implies a variation of typically $\pm$ 10%.

The term "normal" with regard to boiling points, boiling ranges and the like indicates that these values are understood as being corrected for "normal conditions", i.e. a temperature of 25°C and an atmospheric pressure of 1013 mbar. In analogy, the term "normal" with respect to physical states of matter and the like indicates that they are referred to said "normal conditions".

The term "layer" is used herein to refer to any planar or curved stratum having a width dimension that is substantially larger than its thickness dimension; typically, the width:thickness ratio is at least 10:1 and generally well above that value.

In the context of the present invention the term "hydrocarbons" stands for substances consisting of hydrogen and carbon atoms, such as aliphatic hydrocarbons being either saturated, such as alkanes, or unsaturated, such as alkenes and alkynes, and cycloaliphatic being either saturated or unsaturated, and aromatic hydrocarbons. Hydrocarbons obtained by the inventive method generally do not contain pollutants, like sulfur and/or heavy metal elements. This represents a major advantage compared to hydrocarbons produced from petroleum, in particular, when the hydrocarbons are then used as fuel. Since the reservoir of natural gas is much larger compared to the one of petroleum the present invention is therefore very valuable not only from an economic point of view but also taking ecological aspects into consideration.

Sources of gaseous compositions containing carbon dioxide and/or at least one normally gaseous hydrocarbon are for example fermentation gas, coal-bed gas, associated gas from crude oil or natural gas or any waste and exhaust gases deriving from industrial processes and containing carbon dioxide and/or at least one normally gaseous hydrocarbon such as methane, ethane, n-propane or iso-propane. It is, however, in accordance with and within the scope of the present invention to use commercially available methane, ethane, n-propane or iso-propane and carbon dioxide of any purity or any other source of these compounds known to the man skilled in the art.

According to a preferred embodiment of the present invention the at least one normally gaseous hydrocarbon is selected from the group of methane, ethane and propane. It is obvious that a mixture of the aforementioned normally gaseous hydrocarbons and further normally gaseous hydrocarbons can be used for the present invention, as indicated above. Prefereably the normally gaseous composition contains methane.

In a further preferred embodiment of the present invention the carbon dioxide and the methane are contained in the normally gaseous composition at a molar ratio of carbon dioxide:methane of between about 1:1 to about 1:4, preferably between about 1:2 to about 1:3.

The normally solid catalyst is a "shape-selective catalyst" being selected from the group of zeolites, modified zeolites and zeolites-like materials and thus having an at least partially crystalline, typically completely crystalline, solid structure. The term "shape-selective catalyst" is intended to refer to a catalyst that owns a special structure containing channels and pores, respectively, to limit the diffusion of the reacting molecules and the formed product molecules through its framework. Only molecules with diameters smaller than the openings and apertures, respectively, of the channels can pass through the shape-selective catalyst. Moreover, an additional constraint is imposed by the size and shape of the apertures and channels with respect to possible transition states of the reaction.

Zeolites are crystalline aluminosilicates of group IA and group IIA elements such as sodium, potassium, magnesium and calcium with the empirical formula (I)

$$M_{2/n}O \cdot (1-y)Al_2O_3 \cdot ySiO_2 \cdot wH_2O \qquad (I)$$

wherein

n is the valence of the cation M,
y the molar fraction of $SiO_2$ in the zeolite framework, and
w number of water molecules present in the zeolite.

Zeolite structures are based on a three-dimensional framework of $AlO_4$ and $SiO_4$ tetrahedra (Si and Al defined as T atoms) which are interlinked through common oxygen atoms forming O-T-O-T-O bonds to give said three dimensional network through which channels run. Each $AlO_4$ tetrahedron in the framework bears a net negative charge which is compensated by a cation of the aforementioned group IA and group IIA elements. This cation is mobile and can be exchanged by other cations. In the interior of the channels are water molecules and the mentioned mobile cations. Criteria to distinguish zeolites and zeolites structures, respectively, are, inter alia, the number of either T- or O-atoms forming the rings and openings, respectively, controlling diffusion through the channels as well as the molar ratio of $SiO_2/Al_2O_3$, in short the Si/Al ratio.

"Modified zeolites" as used herein refers to natural or synthetic zeolites which have been modified by either cation or ion exchange, thermal treatment, hydrothermal treatment, treatment with inorganic or organic acids, treatment with inorganic or organic F materials, i.e inorganic or organic compounds containing fluor or fluoride, as well as with organic silica materials, and, in particular, by an isomorphic substitution of the T atoms. Such an isomorphic substitution of the T atoms, e.g. leading to silicoaluminophosphates, can be effected particularly by changing the parameters of the zeolite syntheses or by "post synthesis" modifications. Thus, various T atoms can be introduced into a zeolite framework during the synthesis step, such as Ga, Ge, Li, Be, B, Mg, Ti, Mn, Fe, Co, In and As. Such an introduction of a certain element and its concentration in a zeolitic framework usually occurs by mixing the different components in the synthesis gel. However, any other procedure for the introduction of the mentioned and other elements leading to modified zeolites are within the scope of the present invention. Moreover, the preparation method by simply mixing the different components as well as other procedures for the synthesis of modified zeolites are known by the person skilled in the art and need no further explanation herein. In analogy, "post synthesis" modifications of zeolites, i.e. modifications of existing natural or synthetic zeolites to effect a T-site substitution are also within the knowledge of the artisan and do not need to be further explained herein. Thus, various gaseous and liquid reactants as well as different techniques, such as hydrothermal techniques, have been developed for such a post synthesis T-site substitution. In particular, numerous reactants and techniques have been described for aluminium removal with simultaneous substitution by other elements such as Si, Ti, V, Fe, Sn, Ge and P.

"Zeolite-like materials" as used herein refers to normally solid materials, such as aluminophosphates, silicoaluminophosphates, metalloaluminophosphates and metal oxides containing OH groups, which have solid structures similar to zeolites and, therefore, which contain channels with sizes and shapes similar to channel sizes and shapes in zeolite structures. In particular, "zeolite-like materials" refer to crystalline solids which have zeolite-like structures as indicated by their incorporation in the "Atlas of Zeolite Structure Types" reported by Meier and Olson in "Atlas of Zeolites Structure Types", Zeolites 17(1996), 1-230 (report being incorporated herein for all purposes by way of reference). Examples of those isotypic and homeotypic species having zeolite-like structures are reported, in particular, in Table 3 on page 14 of the aforementioned reference.

In accordance with the present invention it has been found that the use of a member selected from zeolites, modified zeolites and zeolite-like materials having at least one 10-ring channel within its structure as the normally solid catalyst for the inventive method limits the growth of the hydrocarbon chain and thus inhibits the undesired formation of solid polymer. Consequently, an increased production and yield, respectively, of liquid fuel, in particular of normally liquid hydrocarbons, results. Moreover, the application of shape-selective catalysts for the inventive method leads to a large amount of branched hydrocarbons, in particular branched normally liquid hydrocarbons, representing a higher-quality fuel.

Typical and preferred materials having a solid structure in accordance with the present invention, and thus having at least one 10-membered ring channel system within the structure, are given in the following by way of their structure type codes consisting of three capital letters. These structure type codes have been set up by the IUPAC Commission on Zeolite Nomenclature and are, inter alia, listed together with their basic structural features and specific examples of zeolites, modified zeolites and zeolite-like materials in the above-mentioned "Atlas of Zeolite Structure Types" reported by Meier and Olson in Zeolites 17(1996), 1-230. Thus, typical normally solid catalysts in accordance with the present invention are of the structure type AEL, AFO, AHT, FER, LAU, MEL, MFI, MFS, MTT and STI.

In a further preferred embodiment of the present invention, the at least one 10-ring channel has a diameter of between about 4 Å and about 7 Å. Such a well-defined pore system further increases the shape-selectivity of the normally solid catalyst since the pores and channels, respectively, must be capable to accommodate the reactants, i. e. the normally gaseous composition, and in particular carbon dioxide and the normally gaseous hydrocarbons or fragments thereof. Moreover, an additional constraint is imposed by the pores and apertures of the channels which control the size and shape of the product molecules. Therefore, the preferred diameter of the at least one 10-ring channel between about 4 Å and about 7 Å according to the invention, leads to a precisely defined arrangement of $AlO_4$ and $SiO_4$ tetrahedra and thus to a shape-selective dielectric barrier discharge reaction with good reproducibility.

In a further preferred embodiment of the present invention the solid structure of the normally solid catalyst have at least two channel systems, wherein at least one channel system is a 10-ring channel. Typically these at least two channel systems are interconnected, wherein, however, the channel intersections are free of super cages and β-cages, respectively, as well as free of α-cages. These super cages and α-cages interconnecting the different channels have diameters which are larger than those of the channels and the apertures of the channels, respectively. Therefore, the preferred embodiment of the invention, in which the solid structure of the normally solid catalyst is free of super cages further increases the shape-selectivity of the transformation by further defining the catalyst.

In accordance with the present invention, the normally solid catalyst has preferably a Si/Al ratio of higher than 6, more preferably higher than 15. As indicated, the Si/Al ratio is a criterion to distinguish different zeolites, modfified zeolites and zeolite-like materials. Moreover, the Si/Al ratio strongly influences important properties of the inventive solid catalyst. Thus, the preferred Si/Al ratio indicated above leads to higher thermal and higher hydrothermal stability of the inventive solid catalyst. This is of particular benefit with respect to the regeneration of the normally solid catalyst since water might be formed during the regeneration process of the catalyst and as a by-product in the inventive dielectric barrier discharge reaction as well as small amounts of carbon deposits and/or polymeric material and hydrocarbons having a carbon chain built by 11 or more carbon atoms, respectively.

The increased hydrothermal stability of the aforementioned preferred embodiments of the present invention thus minimizes and/or avoids damages to the solid catalyst under the reaction conditions caused by the eventual formation of water. Moreover, the preferred Si/Al ratio particularly contributes to the increase of the structure destructive temperature of the inventive normally solid catalysts allowing a standard regeneration taking place at about 600°C. Thus, high silica Zeolite catalysts such as HZSM-5 are thermally stable up to 1000°C.

Preferred normally solid catalysts used in the present invention are a member of the group selected from zeolites and modified zeolites having a crystalline solid structure selected from FER, MEL, MFI, and MTT as specified and defined in the above-mentioned "Atlas of Zeolite Structure Types" reported by Meier and Olson in Zeolites 17 (1996), 1-230. Typical examples of those preferred catalysts are the so-called pentasil-type zeolites, such as Ferrierite and ZSM series catalysts, e.g. ZSM-5, ZSM-23, ZSM-20 and ZSM-11, and the corresponding modified zeolites as defined above.

In particular the use of ZSM-5 zeolites, and here preferably the NaZSM-5 and more preferably the HZSM-5, is highly preferred as the normally solid catalyst for the present invention. The shape-selective Zeolite ZSM-5 is a synthetic high-silica zeolite with a high thermal stability. The MFI-type topology shows a three-dimensional 10-member ring channel system having no super cages in its structure.

The use of a ZSM series zeolite catalyst for the inventive method leads to a product stream containing normally liquid hydrocarbons, as well as syngas, normally gaseous hydrocarbons, and higher hydrocarbons, i.e having a carbon chain of at least 11 carbon atoms (C>11), and/or polymeric material. The products formed in the reaction over ZSM series catalysts, in particular, contain a large amount of branched hydrocarbons having a carbon chain of 6 to 10 carbon atoms improving the octane value and, thus, the quality of gasoline and liquid fuel, respectively. Beneficial for the former is, moreover, the formation of some normally liquid aromatics and oxygenates in the inventive method. The formation of polymer materials and higher hydrocarbons (>C11) is strongly restricted over ZSM catalysts because of their special pore structure and catalytic properties.

The use of zeolite-like materials and, in particular, the use of zeolites and modified zeolites as the normally solid catalyst offers, moreover, the advantage of having high concentrations of OH groups on the surfaces, i.e. on the outer surfaces of the zeolite structure as well as within the zeolite cavities and channels, respectively. In addition to the high concentration of OH groups on zeolite surfaces, an important characteristic of zeolites is the natural coulombic field formed within the zeolite framework. Within this context, it should be noted that both the concentration of OH groups and the strength of the natural coulombic field are controllable and adjustable. Generally, these two features allow the zeolites to easily respond to an external electric field, i.e. the zeolite becomes electrically charged more easily. The control of the dielectric barrier discharge according to the invention allows though to control these charges and electrostatic fields and, therefore, to control zeolite activity and selectivity in the conversion of a normally gaseous composition into a product stream containing at least a normally liquid hydrocarbon.

As already indicated, the use of zeolite-like materials, and in particular zeolites and modified zeolites, as the normally solid catalyst inhibits the formation of carbon black, in particular, the precipitation of carbon black on the surface of the dielectric material, thus, allowing a long term operation of the dielectric-barrier discharge reactor. Moreover, the use of aforementioned catalysts for the inventive method limits the growth of the hydrocarbon chain. Consequently, an increased yield of normally liquid hydrocarbons having a normal boiling range of between about 50°C and about 210°C results and the formation of higher hydrpcarbons (C>11) and polymeric material are suppressed. Furthermore, applying shape-selective catalysts leads to an increased tendency to form branched hydrocarbons, in particularly, of normally liquid branched hydrocarbons representing a high-quality fuel. Additionally, the function of the aforementioned catalysts includes the chemisorption of methane and, thus, the limitation of total decomposition of methane to carbon and hydrogen.

A further advantage of zeolites is the possibility of a controlled incorporation of acid centers in the intracrystalline surface, e.g. during synthesis and/or by subsequent ion exchange reaction. Thus, at temperatures above 300°C, pentasils have acidities comparable to those of mineral acids. Preferably, the normally solid catalyst is an acid type zeolite catalyst, such as HZSM-5 with varying Si/Al ratio as described in Example 1.

In a further preferred embodiment of the invention, the normally solid catalyst comprises at least one substance selected from the group of metal ions and group IA, IIa, IB, IIb and VIII elements of the periodic table. The latter mentioned elements, i.e. alkali, earth alkali elements as well as the elements of the zinc and the copper group and the iron groups of the periodic table can be present either in ionic or atomic form. Those normally solid catalysts are synthesized by procedures generally known to the man skilled in the art, such as any type of ion exchange reactions or impregnations, in case followed by subsequent thermal activation or reduction. Examples of those solid catalysts are the Fe-ZSM-5, Fe/H-ZSM-5, Mo/H-ZSM-5, and H-GaAlMFI. The use of bifunctional catalysts, in particular the introduction of an hydrogenation function in an acidic zeolite, such as the incorporation of $Pt^{2+}$ and $Pd^{2+}$ ions may further prevent or lower the formation of carbon deposits.

The normally liquid fuel obtainable by a dielectric barrier discharge in accordance with the present invention comprises at least 60 mol% of hydrocarbons, typically at least 70 mol% of hydrocarbons, and preferably at least 80 mol% of hydrocarbons having a boiling range of between about 50°C and about 210°C, typically between about 50°C and about 180°C and a ratio branched hydrocarbons:linear hydrocarbons of higher than 6:1, typically about 9:1. The normally liquid fuel generally contains less than 40 mol% of oxygenated hydrocarbons, such as methanol, ethanol or higher, typically branched oxygenates. Typically, the normally liquid fuel comprises less than 30 mol% and preferably less than 20 mol% of oxygenated hydrocarbons. In particular, the selectivity towards methanol is generally less than 2 mol%, typically less than 1 mol% and preferably less than 0.5 mol%.

Further preferred embodiments of the present invention, in particular, of the inventive apparatus are defined in the sub-claims. Thus, a plurality of pairs of first and second electrode means are arranged in an essentially parallel or staked configuration forming a plurality of discharge gaps, the gaps being connected in series to form an elongated path for passage of said normally gaseous composition.

Brief Description of the Drawings

For a better understanding of the nature and scope of the present invention - and not to limit the invention - preferred embodiments and details of the inventive method and apparatus are described in more detail in the following by reference to the drawings, in which:

Fig. 1 is a diagrammatic cross sectional view of a preferred dielectric barrier discharge reactor configuration according to the invention;

Fig. 2 is a diagrammatic cross sectional view of a further preferred dielectric barrier discharge reactor configuration according to the invention;

Detailed Description of the Drawings

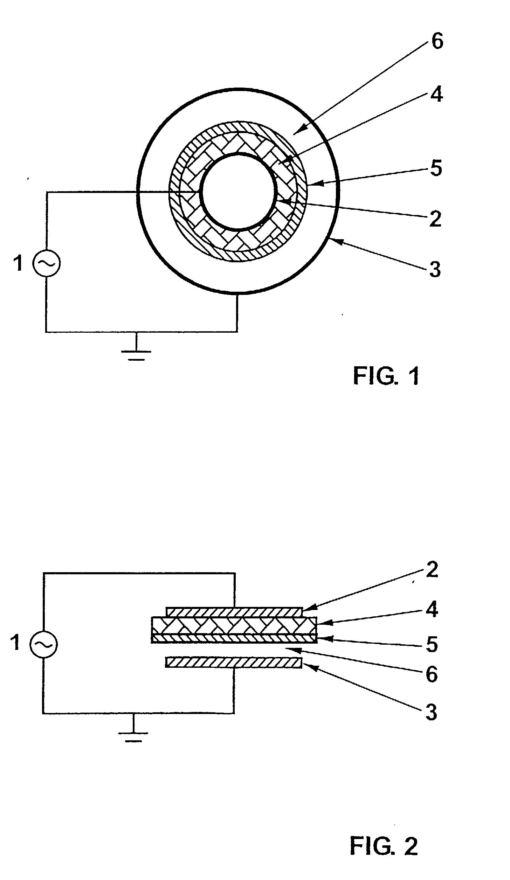

The dielectric barrier discharge is a high pressure non-equilibrium discharge which occurs when alternating voltages are applied to a gas space between two electrodes separated by a non-conducting medium. The amplitude of the alternating high voltage has to be high enough to cause electrical breakdown in the gas space. Fig. 1 shows schematically a cross sectional view of a dielectric barrier discharge reactor according to the invention. The high voltage AC generator 1 is connected to the first electrode 2 and to the second grounded electrode 3 both having an essentially cylindrical form. The electrodes are generally made of corrosion-resistant metals or alloys or of materials covered by at least one layer of an electrically conducting substance. Electrode 2 forms an inner shell and Electrode 3 forms an outer shell. The dielectric layer 4 is typically a glass, quartz or ceramic tube having a thickness of between about 0.1 mm and about 5 mm and covers the effective surface of electrode 2. The shape-selective catalyst 5 shown in Fig. 1, is also formed in essentially cylindrical form and is provided to cover the dielectric layer 4. Typically, the dielectric tube 4 serves as support for the solid catalyst 5. So, the solid catalyst 5, typically in powder form, is disposed in a piece of gas-permeable quartz fleece and wrapped around the outer surface of the dielectric tube 4, i.e. the surface of the dielectric tube 4 facing towards the electrode 3. Further catalyst support arrangements preferably used for the present dielectric barrier discharge reaction are described in EP-899`010 filed by the present assignee (the disclosure of which being incorporated herein for all purposes by way of reference). It is obvious that the form and the size of the solid catalyst, i.e. whether it is applied in powder form or as grains of different sizes and the manner by which the catalyst is supported, i.e by means of the dielectric material and by means of an additional support respectively, can be modified within the scope of the present invention.

The normally gaseous composition passes in the axial direction through the essentially annular discharge gap 6, where it is exposed to the dielectric barrier discharge. The dielectric barrier discharge is effected by an AC potential applied between the first electrode and the second electrode means. The preferred AC potential being in the range of from about 6 kV to about 100 kV and the frequency of the AC potential preferably being in the range of from about 50 Hz to about 1 MHz. Generally a specific electric power up to about 1-25 kW/m$^2$ of electrode area is fed into the discharge reactor by automatically adjusting the amplitude and frequency of the applied voltage. As indicated above, an operating pressure in the range of from about 0.01 bar to about 30 bar, preferably from about 0.1 bar to about 10 bar, at an operating temperature up to about 400°C is maintained in the reactor. The normally gaseous mixture is passed through said reactor preferably at a flow rate of from about 0.1 m$^3$ per hour and m$^2$ of electrode area to about 10 m$^3$ per hour and m$^2$ of electrode area.

When the amplitude of the applied AC electric field reaches a critical value, breakdown is initiated in the gas and a current flows from one electrode to the other. Once breakdown is initiated at any location within the discharge gap, charge accumulates on the dielectric leads to the formation of an opposite electric field. This opposite electric field reduces the external electric field within the gap and interrupts the current flow in a few nanoseconds to form short-lived microdischarges. The duration of the current pulse relates to pressure and properties of gases involved and the dielectrics applied. A large number of such microdischarges will be generated when a sufficiently high AC voltage is applied. The principal advantages of dielectric barrier discharge are that non-equilibrium plasma conditions are established at about atmospheric pressure and that the entire electrode area is effective for discharge reactions.

Fig. 2 shows another preferred configuration of a dielectric barrier discharge reactor according to the invention. The corresponding electrodes, the layer of the normally solid dielectric material and the normally solid catalyst respectively of this embodiment have or are arranged in an essentially planar form. Examples of the dielectric material are glass, as indicated, as well as quartz, ceramics, $ZrO_2$ or $Al_2O_3$.

Further preferred dielectric barrier discharge reactor configurations not being shown in the Figs. 1 and 2 are those, where the solid catalyst either occupies an essential part of the discharge gap 6 or where the solid catalyst covers only a portion of the dielectric material.

Examples

Example 1

The feed gases, i.e. a mixture containing methane and carbon dioxide, were introduced into the system flowing downstream through the reactor. The operative conditions were as follows (cf Table 1): Total flow rate (methane plus carbon dioxide) is 200 ml/min. The molar ratio of methane to carbon dioxide is 1:1. A temperature of 150°C, a pressure of 1 bar, and an electrical power of 500W are applied. The catalysts used are NaZSM-5(38), i.e NaZSM-5 with a molar ratio of $SiO_2/Al_2O_3$ equal to 38, HZSM-5(38), HZSM-5(25)and NaY for comparison. A dielectric barrier discharge is thus initiated. A back pressure valve at the exit of the reactor was used to adjust the pressure. A MTI (Microsensor Technology Inc., M200H) dual-module micro gas chromatograph containing a Poraplot Q column and a molecular sieve 5A-plot column with a TCD detector were used to detect gaseous products. The gas sample was heated by a heated line to avoid possible condensation before it was taken into the gas chormatograph. The liquid sample was analysed by gas chromatography. The amount of higher hydrocarbons (>C11) and polymer materials formed in the dielectric barrier discharge reaction was determined by TGA (Thermogravimetric analysis) measurement. The experiment lasted for 300 min.

The operative conditions as well as the results including the gas phase product distribution are reported in Table 1, wherein the conversion of methane and carbon dioxide respectively are defined as:

$$\text{Conversion } [CH_4] = \{([CH_4]_{in} - [CH_4]_{out})/[CH_4]_{in}\} \times 100\%$$

and

$$\text{Conversion } [CO_2] = \{([CO_2]_{in} - [CO_2]_{out})/[CO_2]_{in}\} \times 100\%$$

respectively. The selectivity of the products are defined as:

$$\text{Selectivity } [prod.] = \{(\text{number of carbon atoms of prod.} \times [prod.]_{out})/\text{total carbon}$$

$$\text{amount converted}\} \times 100\%$$

The analysis of the gas sample reveals formation of carbon monoxide CO, alkanes having 2 to 4 carbon atoms (C2-C4) such as iso-butane, unsaturated hydrocarbons such as ethylene, small amount of oxygenated products such as methanol as well as water and hydrogen.

Table 1.

| Catalyst | NaY | NaZSM-5(38) | HZSM-5(38) | HZSM-5(25) |
|---|---|---|---|---|
| Reaction temperature (K) | 423 | 423 | 423 | 423 |
| Pressure (bar) | 1 | 1 | 1 | 1 |
| $CH_4/CO_2$(molar ratio) | 1 | 1 | 1 | 1 |

Table 1.   (continued)

| Catalyst | NaY | NaZSM-5(38) | HZSM-5(38) | HZSM-5(25) |
|---|---|---|---|---|
| Flow rate (ml/min) | 200 | 200 | 200 | 200 |
| Reaction time(min) | 300 | 300 | 300 | 300 |
| Power applied(W) | 500 | 500 | 500 | 500 |
| Reagents in and out the reactor(g) | | | | |
| $CH_{4in}$ | 21.43 | 21.43 | 21.43 | 21.43 |
| Out | 6.76 | 8.95 | 7.56 | 6.43 |
| Conversion of $CH_4$(wt%) | 68.46 | 58.24 | 64.72 | 69.99 |
| $CO_{2in}$ | 58.93 | 58.93 | 58.93 | 58.93 |
| Out | 35.21 | 38.41 | 36.70 | 32.97 |
| Conversion of $CO_2$(wt%) | 40.25 | 34.82 | 37.72 | 44.05 |
| Gas Product distribution (g) | | | | |
| $H_2$ | 1.32 | 0.84 | 1.40 | 1.59 |
| CO | 16.65 | 16.47 | 18.09 | 19.27 |
| $C_2H_4$ | 0.08 | 0.07 | 0.08 | 0.07 |
| $C_2H_6$ | 2.00 | 1.96 | 2.00 | 1.95 |
| $C_3H_8$ | 1.08 | 0.94 | 1.08 | 1.00 |
| $iso$-$C_4H_{10}$ | 2.11 | 1.70 | 1.90 | 1.83 |
| $C_4H_8$ | 0.20 | 0.25 | 0.17 | 0.24 |
| $C_4H_{10}$ | 0.33 | 0.29 | 0.23 | 0.31 |
| $CH_3OH$ | 0.04 | 0.05 | 0.06 | 0.06 |
| $H_2O$ | 2.73 | 2.38 | 2.69 | 3.04 |
| >$C_5$ and oxygenates and polymer material (g)* | 11.86 | 8.05 | 8.38 | 11.58 |

Table 2 shows the carbon number distribution in the organic liquid phase. The analysis of the liquid sample shows a high yield of gasoline components (C5-C11) being rich in branched hydrocarbons. In particular, the use of ZSM-5 series catalysts lead to a very high selectivity of C5 to C11 hydrocarbons, in particular of C6 to C10. As compared to NaZSM-5(38) the use of the acid-type catalyst HZSM-5(38) having the same Si/Al ratio shifts the selectivity towards longer chain formation and higher hydrocarbons within the aforementioned group, i.e C5-C11 hydrocarbons. Similarly, a higher Si/Al ratio also shifts the selectivity towards the formation of longer chains and higher hydrocarbons, respectively, as indicated by the results of the inventive method obtained with HZSM-5(38) and HZSM-5(25) reported in Table 2.

Table 2.

| Carbon number distribution in liquid organic phase | | | | |
|---|---|---|---|---|
| | NaY | NaZSM-5(38) | HZSM-5(38) | HZSM-5(25) |
| Liquid product composition (wt%) | | | | |
| $C_3$ | 17.86 | 0.12 | 0.16 | 0.24 |
| $C_4$ | 40.51 | 3.40 | 1.76 | 6.13 |
| $C_5$ | 27.66 | 7.99 | 4.99 | 13.82 |
| $C_6$ | 1.65 | 18.62 | 12.54 | 26.68 |
| $C_7$ | 10.21 | 19.29 | 10.93 | 14.70 |

Table 2.   (continued)

| Carbon number distribution in liquid organic phase | | | | |
|---|---|---|---|---|
| | NaY | NaZSM-5(38) | HZSM-5(38) | HZSM-5(25) |
| Liquid product composition (wt%) | | | | |
| $C_8$ | 1.44 | 17.51 | 20.58 | 15.00 |
| $C_9$ | 0 | 24.37 | 24.21 | 16.56 |
| $C_{10}$ | 0 | 7.48 | 19.78 | 5.58 |
| $C_{11}$ | 0.68 | 1.17 | 4.91 | 0.93 |

Table 3.

| Product distribution in liquid organic phase | | | | |
|---|---|---|---|---|
| | NaY | NaZSM-5(38) | HZSM-5(38) | HZSM-5(25) |
| Liquid product distribution(wt%) | | | | |
| Paraffins | 16.80 | 52.39 | 34.02 | 56.99 |
| Aromatics | 0 | 5.57 | 8.27 | 3.28 |
| Naphthenes** | 0.54 | 31.09 | 39.03 | 25.56 |
| Unknowns | 82.66 | 10.95 | 18.68 | 14.17 |

** alicyclic compounds; term often used in the petrochemistry

Table 4.

| TGA Analysis Results | | | |
|---|---|---|---|
| | NaY | NaZSM-5(38) | HZSM-5(25) |
| Pore structure | Three dimensional with interlinking channels | Bidimensional with large intersection (straight and sinusoidal) | Bidimensional with large intersection (straight and sinusoidal) |
| Pore Size (Å) | 7.4(supercage) | 5.4∗5.6 | 5.4∗5.6 |
| Weight loss (wt%) | 39.5 | 21.4 | 15.8 |
| Polymer($>C_{11}$) formed(g)∗ | 1.58 | 0.85 | 0.63 |

∗ after reaction for 300 min.

Table 4 shows the results of the TGA analysis after the inventive dielectric barrier discharge reaction of 300 min. The higher weight loss observed in the case of NaY indicates that, first, NaY has a poorer thermal stability as compared to the ZSM-5 series catalysts NaZSM-5 and HZSM-5, and, second, the use of NaY as the normally solid catalyst for the inventive method leads to much higher amounts of polymeric material or higher hydrocarbons (>C11) as compared to NaZSM-5 and HZSM-5.

The success in the research and development of a feasible utilization of greenhouse gases, in particular methane and carbon dioxide, which led to the present invention signify the attainment of two important objectives: First, slowing down a build-up of greenhouse gases in the atmosphere and, second, better carbon resource utilization. An extra advantage of such an utilization of these major greenhouse gases is the fact that such synthesized liquid fuel does not contain pollutants like sulfur that are usually present in coal and petroleum.

Although certain preferred embodiments of the invention have been described herein, it will be apparent to those skilled in the art to which the invention pertains that modifications and variations of the described embodiments may be made without departing from the spirit and scope of the invention.

List of Reference Signs

1   high voltage AC generator
2   first electrode means
3   second electrode means
4   dielectric layer, dielectric material
5   catalyst
6   discharge gap

**Claims**

1. A method of transforming a normally gaseous composition containing carbon dioxide and at least one normally gaseous hydrocarbon into a product stream containing at least one normally liquid hydrocarbon; said method comprising the steps of:

   feeding said composition into a reactor including a first electrode means, a second electrode means and at least one layer of a normally solid dielectric material positioned between said first and said second electrode means;
   submitting said composition within said reactor in the presence of a normally solid catalyst to a dielectric barrier discharge, wherein said normally solid catalyst is a member selected from the group of zeolites, modified zeolites and zeolite-like materials, and wherein said normally solid catalyst has an at least partially crystalline solid structure with at least one 10-ring channel; and
   controlling said dielectric barrier discharge to convert said normally gaseous composition into said product stream.

2. The method of claim 1 wherein said at least one normally gaseous hydrocarbon is selected from the group of methane, ethane and propane.

3. The method of claim 1 or 2 wherein said at least one normally gaseous hydrocarbon is methane.

4. The method of claim 2 or 3 wherein said carbon dioxide and said methane are contained in said normally gaseous composition at a molar ratio of carbon dioxide:methane of between about 1:1 to about 1:4, preferably between about 1:2 to about 1:3.

5. The method of any of claims 1 to 4 wherein said at least one 10-ring channel has a diameter of between about 4 Å and about 7 Å.

6. The method of any of claims 1 to 5 wherein said solid structure of said normally solid catalyst is free of super cages.

7. The method of any of claims 1 to 6 wherein said normally solid catalyst has a Si/Al ratio of higher than 6, preferably higher than 15.

8. The method of any of claims 1 to 7 wherein said normally solid catalyst is a zeolite or a modified zeolite having a crystalline solid structure selected from FER, MEL, MFI, and MTT.

9. The method of any of claims 1 to 8 wherein said normally solid catalyst comprises at least one substance selected from the group of metal ions and group IA, IIa, IB, IIb and VIII elements of the periodic table.

10. A normally liquid fuel obtainable by a dielectric barrier discharge, said normally liquid fuel comprising at least 60 mol% of hydrocarbons having a normal boiling range of between about 50°C and about 210°C, and less than 40 mol% of oxygenated hydrocarbons.

11. An apparatus for transforming a normally gaseous composition containing carbon dioxide and at least one normally gaseous hydrocarbon into a product stream containing at least one normally liquid hydrocarbon; said apparatus comprising:

a dielectric barrier discharge reactor including a first electrode means, a second electrode means and at least one layer of a normally solid dielectric material positioned between said first and said second electrode means; means for applying an AC potential between said first and said second electrode means;

means for feeding said composition into said reactor;

a normally solid catalyst arranged for contact with said gaseous composition when subjected to said dielectric barrier discharge, wherein said normally solid catalyst is a member selected from the group of zeolites, modified zeolites and zeolite-like materials, and wherein said normally solid catalyst has an at least partially crystalline solid structure with at least one 10-ring channel; and

means for controlling said dielectric barrier discharge to convert said normally gaseous composition into said product stream.

12. The apparatus of claim 11 wherein said at least one normally gaseous hydrocarbon is selected from the group of methane, ethane and propane, preferably said at least one normally gaseous hydrocarbon being methane.

13. The apparatus of claim 12 wherein said carbon dioxide and said methane are contained in said normally gaseous composition at a molar ratio of carbon dioxide:methane of between about 1:1 to about 1:4, preferably between about 1:2 to about 1:3.

14. The apparatus of any of claims 11 to 13 wherein said at least one 10-ring channel has a diameter of between about 4 Å and about 7 Å.

15. The apparatus of any of claims 11 to 14 wherein said solid structure of said normally solid catalyst is free of super cages.

16. The apparatus of any of claims 11 to 15 wherein said normally solid catalyst has a Si/Al ratio of higher than 6, preferably higher than 15.

17. The apparatus of any of claims 11 to 16 wherein said normally solid catalyst is a zeolite or a modified zeolite having a crystalline solid structure selected from FER, MEL, MFI, and MTT.

18. The apparatus of any of claims 11 to 17 wherein said normally solid catalyst comprises at least one substance selected from the group of metal ions and group IA, IIa, IB, IIb and VIII elements of the periodic table.

FIG. 1

FIG. 2

**European Patent Office**

# EUROPEAN SEARCH REPORT

Application Number

EP 00 81 0248

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | DE 42 20 865 A (ASEA BROWN BOVERI) 18 February 1993 (1993-02-18)<br><br>* claims 1,7,11 *<br>* figures 1-3 *<br>--- | 1,2,5, 7-9,11, 12,14, 16-18 | C07C2/86 B01J19/08 |
| Y | WO 97 45386 A (EXXON CHEMICAL PATENTS INC) 4 December 1997 (1997-12-04)<br><br>* claims 1,7,10,11,17 *<br>* page 17, line 23 - page 18, line 7 *<br>--- | 1,2,5, 7-9,11, 12,14, 16-18 | |
| A | DD 242 007 A (AKAD WISSENSCHAFTEN DDR) 14 January 1987 (1987-01-14)<br>----- | | |

TECHNICAL FIELDS SEARCHED (Int.Cl.7)

C07C
B01J

The present search report has been drawn up for all claims

| Place of search | Date of completion of the search | Examiner |
|---|---|---|
| THE HAGUE | 30 June 2000 | De Herdt, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**EP 1 038 855 A1**

## ANNEX TO THE EUROPEAN SEARCH REPORT
## ON EUROPEAN PATENT APPLICATION NO.

EP 00 81 0248

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2000

| Patent document cited in search report | | Publication date | Patent family member(s) | | Publication date |
|---|---|---|---|---|---|
| DE 4220865 | A | 18-02-1993 | NONE | | |
| WO 9745386 | A | 04-12-1997 | AU | 3228997 A | 05-01-1998 |
| | | | AU | 4038597 A | 05-01-1998 |
| | | | BR | 9709620 A | 10-08-1999 |
| | | | BR | 9709622 A | 10-08-1999 |
| | | | CA | 2254893 A | 04-12-1997 |
| | | | CA | 2255873 A | 04-12-1997 |
| | | | EP | 0912473 A | 06-05-1999 |
| | | | EP | 0914311 A | 12-05-1999 |
| | | | WO | 9745387 A | 04-12-1997 |
| | | | US | 5998686 A | 07-12-1999 |
| | | | US | 5994603 A | 30-11-1999 |
| DD 242007 | A | 14-01-1987 | NONE | | |

EPO FORM P0459

For more details about this annex : see Official Journal of the European Patent Office, No. 12/82

15